# EUROPEAN PATENT SPECIFICATION

(11) **EP 3 998 025 B1**
(45) Date of publication and mention of the grant of the patent: **08.11.2023**
(21) Application number: 20932836.8
(22) Date of filing: 12.10.2020
(51) Int. Cl.: A61B 17/04

(54) **SUTURING DEVICE FOR USE IN DEEP CHANNEL**
NÄHVORRICHTUNG ZUR VERWENDUNG IN EINEM TIEFEN KANAL
DISPOSITIF DE SUTURE DESTINÉ À ÊTRE UTILISÉ DANS UN CANAL PROFOND

(30) Priority: 18.09.2020 CN 202010987120
(43) Date of publication of application: 18.05.2022
(73) Proprietor: 2020 (Beijing) Medical Technology Co., Ltd., Beijing 100176 (CN)
(72) Inventor: WANG, Daiwen, Beijing 100176 (CN); YIN, Heping, Beijing 100176 (CN); WANG, Linzhong, Beijing 100176 (CN)
(74) Representative: Sach, Greg Robert
(86) International application number: PCT/CN2020/120285
(87) International publication number: WO 2022/056969

(56) References cited:
- EP-A2- 0 643 945
- WO-A1-98/07374
- WO-A1-2015/193317
- AU-A1- 2008 229 746
- CN-A- 103 079 479
- CN-A- 105 943 101
- CN-A- 109 171 858
- CN-U- 201 578 299
- CN-U- 205 054 315
- CN-U- 208 573 778
- CN-U- 213 249 393
- US-A1- 2008 091 220

## Description

### TECHNICAL FIELD

The present disclosure relates to the field of surgical instruments, in particular to a single needle suture device suitable for deep channel suture.

### BACKGROUND ART

When tissues such as skin and bones are injured and broken or some operations performed inside the skin are completed, it is often necessary to suture the wound to repair the injury. The suture process is often carried out by a single needle suture device, which can avoid the suture needle from scratching the tissue during shuttling back and forth of the suture needle in the tissue. To some extent, the suture device frees the hands of medical staff and improves the efficiency of suture. However, traditional suture devices usually suture shallow wounds. When the wound is deeper, the suture is more difficult and not easy to operate.

From document WO 2015/193317 a suture device for a deep channel is known, the device comprising a casing and a suture assembly, a first suture rod and a second suture rod, wherein the suture assembly comprises a suture hooking rod, a puncture needle, a first push needle and a second push needle, a top end of the suture hooking rod being provided with a bending portion. The puncture needle is of a hollow tubular structure with a top end opened, the first push needle and the second push needle being both arranged in the hollow tubular structure of the puncture needle, with the first push needle being configured to push the first suture rod and the second push needle being configured to push the second suture rod.

### SUMMARY

In order to solve the defects in the prior art, the present invention provides a suture device which can realize suturing function in a deep channel

It is provided a suture device, including a casing and a suture assembly.

The suture assembly includes a suture hooking rod, a puncture needle, a first push needle and a second push needle.

The suture hooking rod is a hollow structure with two ends opened, a top end of the suture hooking rod is provided with a bending portion, the bending portion is inverted L-shaped, and a transverse portion of a top end of the bending portion is provided with a hole for the puncture needle to pass through.

The puncture needle is of a hollow tubular structure with a top end opened, and a side of the puncture needle is further provided with a first opening.

The puncture needle is arranged in the hollow structure of the suture hooking rod.

The first push needle and the second push needle are both arranged in the hollow tubular structure of the puncture needle, the first push needle is configured to push the first suture rod, and the second push needle is configured to push the second suture rod.

Further, the second push needle is of a hollow tubular structure, and a top end of the second push needle is configured to push against a second suture rod, and the second suture rod is of a hollow tubular structure

The first push needle is inserted into the hollow structures of the second push needle and the second suture rod, and a top end of the first push needle is configured to push against the first suture rod.

Further, a pinhead of the puncture needle is of a segmented structure, and comprises a first needle segment and a second needle segment, the first needle segment has a first extension surface, and the second needle section has a second extension surface; a longitudinal length of the first needle segment is less than or equal to a longitudinal length of the second needle segment, and an angle formed between a connecting line through two longitudinal end points in the first extension surface and a horizontal plane is greater than an angle formed between a connecting line through two longitudinal end points in the second extension surface and the horizontal plane.

Further, the suture device further includes a puncture needle control component, the puncture needle control component includes a knob, a gear and a rack, the rack is connected to the puncture needle, and the knob drives the rack to move by the gear.

Further, the suture device further includes a first push needle control component and a second push needle control component.

The first push needle control component includes a first slider, a first push button and a first slide rail arranged on the first slider, and the first push button is connected with the first push needle.

The second push needle control component includes a second slider, a second push button and a second slide rail arranged on the second slider, and the second push button is connected with the second push needle.

Further, the first slide rail and the second slide rail are provided with elastic components for returning the first push button and the second push button to corresponding initial positions quickly, respectively.

Further, a groove is further provided on a side of the suture hooking rod, and the groove corresponds to the first opening.

Further, the suture assembly further includes a suture tube, and the suture tube is arranged behind the groove.

Further, the suture assembly further includes a receiving tube. The receiving tube is arranged in the suture hooking rod and is of a hollow tubular structure for accommodating the puncture needle, and a second opening is provided on a side of the receiving tube. The groove and the first opening correspond to the second opening.

Further, a handle is provided on the casing.

The beneficial effects of the present disclosure are provided as follows.
1) The present disclosure can push the auxiliary suture tools at both ends of the sutures into two sides of a wound to be sutured by arrangement of two push needles; and furthermore, by providing a bending hook at the top end of the suture hooking rod, the auxiliary suture tools can be hooked out of the inner side of the wound; a knot can be made outside the wound, which is conducive to recovery of the wound after suturing while reducing difficulty of operation.
2) By arranging the handle on the casing of the suture device, the suture device can be conveniently held during the operation. Providing the elastic parts on the push button slide rail connected with the push needle, can make the push needle rebound quickly. In actual operation, it saves time and effort, and greatly improves efficiency.
3) The pinhead of the puncture needle of the present disclosure adopts a double-needle segment structure. Compared with the traditional single-needle segment structure, this structure is less harmful to the human body during puncture.
4) Through arrangement of the second needle segment of the pinhead on the puncture needle, the puncture resistance can be greatly reduced without reducing strength of the needle. The puncture needle can be used for puncturing harder tissues of the human body. Furthermore, a length of the second needle segment can be appropriately increased so that the pinhead can have sufficient strength and is not easy to break.
5) Since the suture device only extends the bending portion into the suture opening, it is convenient to extend and take out the bending portion, and an external knotting can be realized. Therefore, the suture device can also be used within a channel with a length of more than 300 mm, depending on a size of the bending portion of the suture device.

### BRIEF DESCRIPTION OF THE DRAWINGS

Fig. 1 is a cross-sectional view of a suture assembly of a suture device according to the present disclosure.
Fig. 2 is an enlarged view of part I in Fig. 1.
Fig. 3 is a schematic view showing a structure of a second opening of a receiving tube of the suture device according to the present disclosure.
Fig. 4 is a schematic diagram showing a front part structure of an outside surface of a suture hooking rod provided with a groove, of the suture device according to the present disclosure.
Fig. 5 is a schematic diagram of an internal structure of the suture device
Fig. 6 is a schematic diagram of an external structure of the suture device
Fig. 7 is a schematic diagram showing a longitudinal structure of a pinhead of a puncture needle of the suture device, when the pinhead is placed horizontally.
Fig. 8 is a schematic diagram showing a lateral structure of the pinhead of the puncture needle of the suture device, when the pinhead is placed horizontally.

List of reference numbers: 1 casing, 11 handle, 21 suture hooking rod, 22 puncture needle, 221 first extension surface, 222 second extension surface, 23 first push needle, 24 second push needle, 25 suture tube, 26 bending portion, 27 hole, 28 receiving tube, 29 groove, 31 knob, 32 gear, 33 rack, 41 first slider, 42 first push button, 43 first slide rail, 51 second slider, 52 second push button, 53 second slide rail.

### DETAILED DESCRIPTION OF THE EMBODIMENTS

The technical solutions in the embodiments of the present disclosure will be clearly and completely described below in conjunction with the accompanying drawings in the specification of the present disclosure. Apparently, the described embodiments are only a part of the embodiments of the present disclosure, rather than all the embodiments. Based on the embodiments of the present disclosure, all other embodiments obtained by those of ordinary skill in the art without creative work shall fall within the protection scope of the present disclosure.

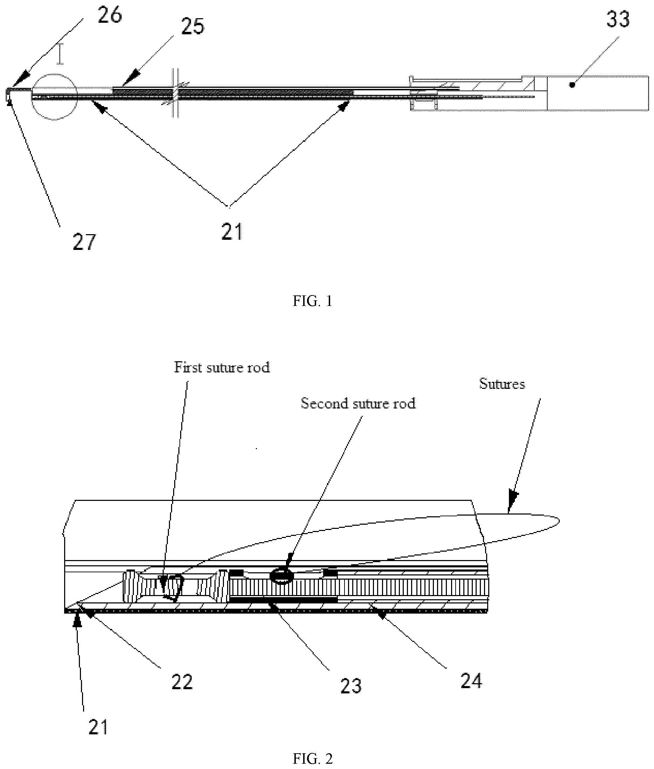

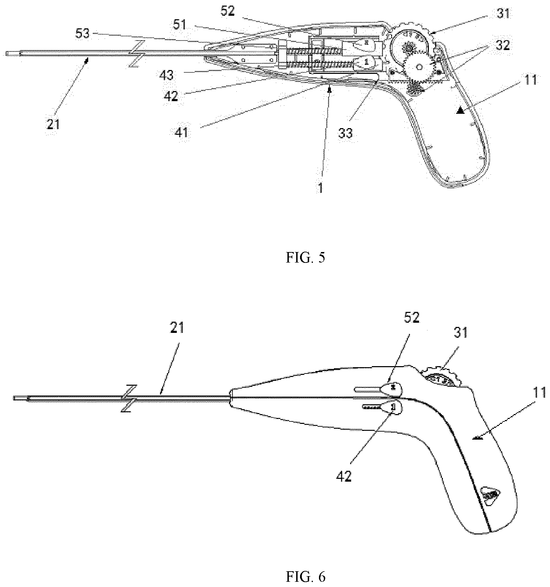

Referring to Figs. 1-8, it is provided a suture device. The suture device includes a casing1 and a suture assembly. The suture assembly includes a suture hooking rod 21, a puncture needle 22, a first push needle 23, and a second push needle 24. The suture hooking rod 21 is of a hollow structure opened at both ends. A top end of suture hooking rod 21 is provided with a bending portion 26 which is inverted L-shaped, and a transverse part of the top end is formed with a hole for the puncture needle to pass through, the hole is convenient to push a suture rod out of the top end of the bending portion 26. The puncture needle 22 is arranged in the hollow structure of the suture hooking rod 21. The puncture needle 22 is of a hollow tubular structure with an open top, and a first opening is provided at a side thereof. The first push needle 23 and the second push needle 24 are both arranged in the hollow structure of the puncture needle 22. The first push needle 23 is used to push the first suture rod, and the second push needle 24 is used to push the second suture rod.

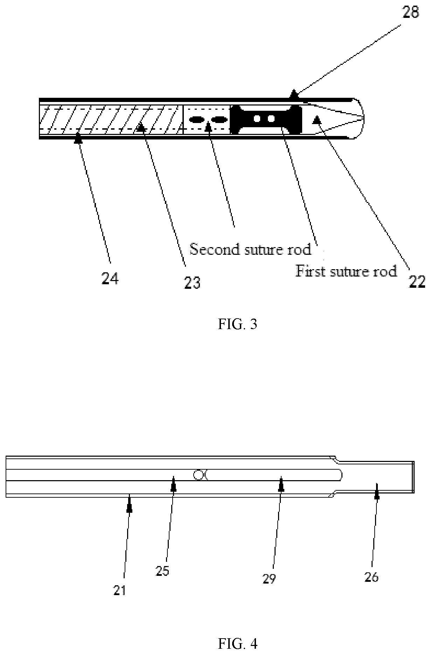

In a preferred embodiment of the present disclosure, arrangement of the first push needle 23 and the second push needle 24 in the hollow structure of the puncture needle 22 is shown in Fig.2. A second push needle 24 is formed as a tubular structure with two open ends. A top end of the second push needle is used to push against the second suture rod. The first push needle is inserted in the hollow structures of the second push needle and of the second suture rod, and a top end of the first push needle is used to push against the first suture rod. Two ends of adapted sutures are connected to the first suture rod and the second suture rod respectively. The second suture rod is of a hollow annular structure. The first suture rod is located in front of the second suture rod and is sized to block advancement of the second suture rod. Preferably, the first suture rod may be a solid suture rod, and an outer diameter of the first suture rod is greater than or equal to an outer diameter of the second suture rod to block advancement of the second suture rod. When in use, the second suture rod is sleeved outside the first push needle 23 first, with a bottom end of the second suture rod 24 against the second push needle 24, and then the first suture rod is arranged above the second suture rod, with a bottom end of the first suture rod 23 against the first push needle 23, which can ensure that the second suture rod is not affected during pushing the first suture rod out by the first push needle. In order to further explain the structure and arrangement, Fig. 3 is a schematic diagram showing the arrangement viewed from the second opening of the receiving tube 28. The two holes on the first suture rod and the second suture rod are used to tie the sutures, and the position of the first push needle 23 inside the second push needle 24 and the second suture rod is shown by the dotted lines.

The present disclosure may also include a puncture needle control component. The puncture needle control component may include a knob 31, a gear 32, and a rack 33. The rack 33 is connected to the puncture needle 22. The knob 31 drives the rack 33 to move through the gear 32, so the puncture needle 22 is controlled by the knob 31 to move. For example, by turning the knob 31 forward, the puncture needle 22 can extend out of the hooking rod 21, pierce the tissue to be sutured, and pass through the hole 27 at the top end of the bending portion. By turning the knob 31 backward, the puncture needle 22 can be retracted into the suture hooking rod 21, and at the same time, the knob 31 can be partially exposed outside the casing 1 for convenience of use.

Further, the present disclosure may also include a first push needle control component and a second push needle control component. The first push needle control component may include a first slider 41, a first push button 42 provided on the first slider 41 and the first slide rail 43. The first push button 42 is connected to the first push needle 23. Therefore, by pushing the first push button 42 forward, the first slider 43 can be moved forward on the first slide rail 43, and at the same time it can drive the first push needle 23 to move forward. For example, when the puncture needle 22 passes through the hole 27 at the top end of the bending portion, by pushing the first push button 42 to slide forward, the first push needle 23 can extend out of the puncture needle 22. When the first suture rod is placed on the first push needle 23 (as shown in Fig. 2), and the first push needle 23 extends out of the puncture needle 22, the first suture rod is pushed out of the puncture needle 22 and kept outside the hole 27. Therefore, by pushing the first push button 42 backward, the first push needle 23 can be moved to an initial position.

The second push needle control component can also be configured as the same as the first push needle control component, and it can include a second slider 51, a second push button 52 provided on the second slider 51, and a second slide rail 53. The second push button 52 is connected to the second push needle 24, so movement of the second push button 52 on the second slide rail can drive movement of the second push needle 24. For example, when the puncture needle 22 passes through the hole 27 at the top end of the bending portion, by pushing the second push button 42 to slide forward, the second push needle 23 can extend out of the puncture needle 22. When the second suture rod at the other end of the sutures is placed on the second push needle 24, and the second push needle 24 extends out of the puncture needle 22, the second suture rod is pushed out of the puncture needle 22 and kept outside the hole 27. When the second push button 52 is pushed backward, the second push needle 24 can be moved to its initial position.

After the first suture rod and the second suture rod are pushed out of the hole 27, the bending portion 26 is taken out from the sutured place, and the first suture rod and the second suture rod are both taken out of the sutured place. No suture rod is inside the sutured place, then a knot can be tied outside the sutured place, which reduces difficulty of the operation and is also conducive to recovery of the sutured wound.

In the present disclosure, the puncture needle control component can also be configured as a combination structure of a slider and a push button, and the first push needle control component and the second push needle control component can also be configured as a combination structure of a knob, a gear, and a rack, as long as components can control extension and retraction of the puncture needle 22, the first push needle 23 and the second push needle 24, they are all applicable to this disclosure.

Furthermore, the first slide rail 43 of the first push needle control component and the second slide rail 53 of the second push needle control component can also be provided with elastic components, such as springs, to quickly reset the first push button and the second push button. The purpose of arrangement of the elastic components is convenient to use during the operation and save suturing time. Furthermore, for convenience of use, the first push button and the second push button can be arranged outside of the casing.

In order to be more convenient to use during the operation, a handle, such as a pistol-type handle, can be provided on the casing, which is more convenient to hold.

Further, in a preferred embodiment, as shown in FIG. 4, a groove 29 may be provided on a side surface of the suture hooking rod 21, and the groove 29 corresponds to the first opening, the suture can be organized and received in the groove outside the suture hooking rod 21 so as to prevent knotting of the sutures. At the same time, to ensure that the suture at the end of the second suture rod is not cut by the first opening when the puncture needle 22 punctures, a length of the first opening on the puncture needle 22 can be calculated according to actual strokes of the push needle and the puncture needle.

In order to facilitate storage of the exposed sutures, as shown in FIG. 4, a suture tube 25 can also be provided behind the groove 29 of the suture hooking rod 21.

Because the puncture needle is in close contact with the tissue during the puncture process, high cleanliness of the puncture needle is required. In addition to disinfection during use, in the storage process, in order to protect the puncture needle 22 and reduce pollution to the puncture needle 22 caused by the external, the suture device is also provided with a receiving tube 28, which is provided in the suture hooking rod 21, for receiving the puncture needle 22. That is, the puncture needle 22 is provided in the receiving tube 28, and the receiving tube 28 is provided in the suture hooking rod 21. As shown in Fig. 3, the receiving tube 28 is of a hollow tubular structure with a second opening on the side, and the groove 29, the first opening, and the second opening correspond to each other.

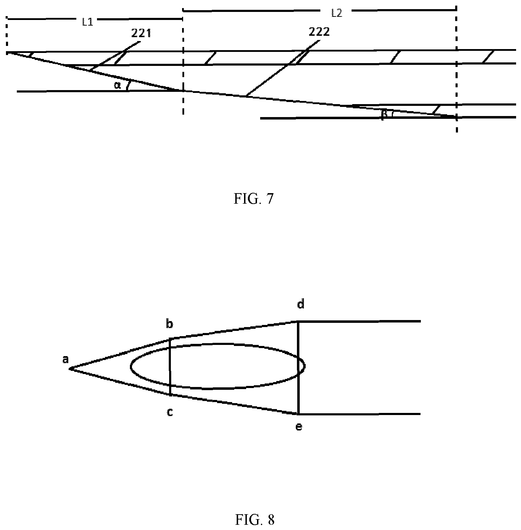

Further, in a preferred embodiment, as shown in Figs. 7 to 8, the pinhead of the puncture needle 22 is preferably a segmented structure, and includes a first needle segment and a second needle segment. The first needle segment has a first extension surface 221, the second needle segment has a second extension surface 222, and a longitudinal length of the first needle segment (a vertical distance from the front end to the rear end of the first needle segment, as shown by L1 in Fig. 7) is less than or equal to a longitudinal length of the second needle segment (a vertical distance from the front end to the back end of the second needle segment, as shown by L2 in Figure 7). An angle (i.e. the angle α in Fig.7) formed between a longitudinal centerline of the first extension surface (a connecting line through an apex of the first extension surface and a center of the bottom end, such as the connecting line through a point A and a midpoint of the line segment BC, as shown in Fig. 8) and a horizontal plane is greater than an angle formed between a longitudinal centerline of the second extension surface (a connecting line through a center of the top end and the center of the bottom end of the second extension surface, such as the connecting line through a midpoint of the line segment BC and a midpoint of the line segment DE, as shown in Fig.8) and the horizontal plane (i.e. the angle β in Fig. 7).

By configuring the angle between the connecting line through the two longitudinal end points of the first extension surface and the horizontal plane to be greater than the angle between the connecting line through the two longitudinal end points of the second extension surface and the horizontal plane, it can be ensured that the second needle segment has a smaller inclination than the first needle segment. When a traditional single-needle segment structure punctures, and the entire needle segment enters the tissue, which will cause greater damage to the tissue. When the present disclosure is used, after the first needle segment completely pierces into the tissue, when the second needle segment pierce into the tissue, due to the second needle segment with a smaller inclination to a certain extent, an increasing amplitude of a pierce width may be reduced, which reduces a pierce area and has a small wound, thereby having reduced damage to the tissue. Providing the second needle segment may reduce addition of the width of the pinhead. Compared with a traditional single-needle segment pinhead, the width of the double-needle segment pinhead with the same length as the single-needle segment pinhead is smaller, which may cause less damage to the tissue during puncture. Under the same widest width, the double-needle segment pinhead can be made longer than the single-needle segment pinhead, which has less resistance and is more suitable for puncturing harder tissues of the human body.

It should be noted that the suture rod referred here is an auxiliary tool in suture, and other types of auxiliary tools are applicable as long as they meet the requirements, and there is no restriction.

The specific operation process will be further introduced below in conjunction with the embodiments.

### Example 1

a. Extending the bending portion 26 into the tissue to be sutured, turning the knob 31 forward to push the puncture needle 22 out of the receiving tube 28 and the suture hooking rod 21 to pierce the tissue to be sutured and pass through the hole 27, and stopping turning the knob 31;
b. Pushing the first push button 42 forward to drive the first push needle 23 to push the first suture rod out of the puncture needle 22, releasing the first push button 42, where the spring drives the first push button 42 to reset, and the first push needle 23 is returned to its initial position at the same time;
c. Turning the knob 31 backward to return the puncture needle 22 to its initial positon, where the first suture rod stays outside the top end of the hole 27;
d. Turning the suture device to the other side of the tissue to be sutured, turning the knob 31 forward to push the puncture needle 22 out of the receiving tube 28 and the suture hooking rod21 to pierce the tissue to be sutured and pass through the hole 27, and stopping turning the knob 31;
e. Pushing the second push button 52 forward to drive the second push needle 24 to push the second suture rod out of the puncture needle 22, releasing the second push button 52, where the spring drives the second push button 52 to return to its initial position, and the second push needle 24 is returned to its initial position at the same time;
f. Turning the knob 31 backward to return the puncture needle 22 to its initial position, where the second suture rod stays outside the top end of the hole 27;
g. Taking out the bending portion 26 from the sutured place, where the first suture rod and the second suture rod are taken out of the inner side of the sutured place; and the first suture rod, the second suture rod and the excess sutures are cut off; making a knot, and completing the suture.

In summary:
1) The present disclosure can push the auxiliary suture tools at both ends of the sutures into two sides of a wound to be sutured by arrangement of two push needles; and furthermore, by providing a bending hook at the top end of the suture hooking rod, the auxiliary suture tools can be hooked out of the inner side of the wound; a knot can be made outside the wound, which is conducive to recovery of the wound after suturing while reducing difficulty of operation.
2) By arranging the handle on the casing of the suture device, the suture device can be conveniently held during the operation. Providing the elastic parts on the push button slide rail connected with the push needle, can make the push needle rebound quickly. In actual operation, it saves time and effort, and greatly improves efficiency.
3) The pinhead of the puncture needle of the present disclosure adopts a double-needle segment structure. Compared with the traditional single-needle segment structure, this structure is less harmful to the human body during puncture.
4) Through arrangement of the second needle segment of the pinhead on the puncture needle, the puncture resistance can be greatly reduced without reducing strength of the needle. The puncture needle can be used for puncturing harder tissues of the human body. Furthermore, a length of the second needle segment can be appropriately increased so that the pinhead can have sufficient strength and is not easy to break.
5) Since the suture device only extends the bending portion into the suture opening, it is convenient to extend and take out the bending portion, and an external knotting can be realized. Therefore, the suture device can also be used within a channel with a length of more than 300 mm, depending on a size of the bending portion of the suture device.

## Claims

1. A suture device for a deep channel, comprising a casing (1) and a suture assembly; wherein the suture assembly comprises a suture hooking rod (21), a puncture needle (22), a first push needle (23) and a second push needle (24);
wherein the suture hooking rod (21) is of a hollow structure with two ends opened, a top end of the suture hooking rod is provided with a bending portion (26), the bending portion (26) is inverted L-shaped, and a transverse portion of a top end of the bending portion is provided with a hole (27) for the puncture needle (22) to pass through;
the puncture needle (22) is of a hollow tubular structure with a top end opened, and a side of the puncture needle (22) is further provided with a first opening;
the puncture needle (22) is arranged in the hollow structure of the suture hooking rod (21);
the first push needle (23) and the second push needle (24) are both arranged in the hollow tubular structure of the puncture needle (22), the first push needle (23) is configured to push the first suture rod, and the second push needle (24) is configured to push the second suture rod.

2. The suture device according to claim 1, wherein the second push needle (24) is of a hollow tubular structure, and a top end of the second push needle is configured to push against a second suture rod, and the second suture rod is of a hollow tubular structure;
the first push needle (23) is inserted into the hollow structures of the second push needle (24) and the second suture rod, and a top end of the first push needle is configured to push against the first suture rod; the first suture rod is provided in front of the second suture rod, and an outer diameter of the first suture rod is greater than an inner diameter of the second suture rod to block advancement of the second suture rod.

3. The suture device according to claim 1, wherein a pinhead of the puncture needle (22) is of a segmented structure, and comprises a first needle segment and a second needle segment, the first needle segment has a first extension surface (221), and the second needle section has a second extension surface (222); a longitudinal length of the first needle segment is less than or equal to a longitudinal length of the second needle segment, and an angle formed between a longitudinal centerline of the first extension surface (221) and a horizontal plane is greater than an angle formed between a longitudinal centerline of the second extension surface (222) and the horizontal plane.

4. The suture device according to claim 1, further comprising a puncture needle control component, the puncture needle control component comprises a knob (31), a gear (32) and a rack (33), the rack (33) is connected to the puncture needle (22), and the knob (31) drives the rack (33) to move by the gear (32).

5. The suture device according to claim 1 or claim 4, further comprising a first push needle control component and a second push needle control component;
the first push needle control component comprises a first slider (41), a first push button (42) and a first slide rail (43) arranged on the first slider (41), and the first push button (42) is connected with the first push needle (23);
the second push needle control component comprises a second slider (51), a second push button (52) and a second slide rail (53) arranged on the second slider (51), and the second push button is connected with the second push needle (24).

6. The suture device according to claim 5, wherein the first slide rail (43) and the second slide rail (53) are provided with elastic components for returning the first push button (42) and the second push button (52) to corresponding initial positions quickly, respectively.

7. The suture device according to claim 1, wherein a groove (29) is further provided on a side of the suture hooking rod (21), and the groove (29) corresponds to the first opening.

8. The suture device according to claim 7, wherein the suture assembly further comprises a suture tube (25), and the suture tube (25) is arranged behind the groove (29).

9. The suture device according to claim 7, wherein the suture assembly further comprises a receiving tube (28), the receiving tube (28) is arranged in the suture hooking rod (21) and is of a hollow tubular structure for accommodating the puncture needle (22), and a second opening is provided on a side of the receiving tube; the groove (29) and the first opening correspond to the second opening.

10. The suture device according to claim 1, wherein a handle (1) is provided on the casing (1).

## Patentansprüche

1. Nahtvorrichtung für einen tiefen Kanal, die ein Gehäuse (1) und eine Nahtanordnung aufweist; wobei die Nahtanordnung einen Nahthakenstab (21), eine Punktionsnadel (22), eine erste Drucknadel (23) und eine zweite Drucknadel (24) aufweist;
wobei der Nahthakenstab (21) eine hohle Struktur mit zwei offenen Enden aufweist, ein oberes Ende des Nahthakenstabs mit einem Biegeabschnitt (26) versehen ist, der Biegeabschnitt (26) umgekehrt L-förmig ist, und ein Querabschnitt eines oberen Endes des Biegeabschnitts mit einem Loch (27) versehen ist, durch das die Punktionsnadel (22) hindurchgehen kann;
die Punktionsnadel (22) eine hohle röhrenförmige Struktur mit einem offenen oberen Ende hat, und eine Seite der Punktionsnadel (22) außerdem mit einer ersten Öffnung versehen ist;
die Punktionsnadel (22) in der hohlen Struktur des Nahthakenstabes (21) angeordnet ist;
die erste Drucknadel (23) und die zweite Drucknadel (24) beide in der hohlen röhrenförmigen Struktur der Punktionsnadel (22) angeordnet sind, die erste Drucknadel (23) dazu eingerichtet ist, den ersten Nahtstab zu schieben, und die zweite Drucknadel (24) dazu eingerichtet ist, den zweiten Nahtstab zu schieben.

2. Nahtvorrichtung nach Anspruch 1, wobei die zweite Drucknadel (24) eine hohle röhrenförmige Struktur aufweist und ein oberes Ende der zweiten Drucknadel dazu eingerichtet ist, gegen einen zweiten Nahtstab zu drücken, und der zweite Nahtstab eine hohle röhrenförmige Struktur aufweist;
die erste Drucknadel (23) in die hohlen Strukturen der zweiten Drucknadel (24) und des zweiten Nahtstäbchens eingeführt ist und ein oberes Ende der ersten Drucknadel dazu eingerichtet ist, gegen den ersten Nahtstab zu drücken; der erste Nahtstab vor dem zweiten Nahtstab vorgesehen ist und ein Außendurchmesser des ersten Nahtstabes größer als ein Innendurchmesser des zweiten Nahtstabes ist, um das Vorrücken des zweiten Nahtstabes zu blockieren.

3. Nahtvorrichtung nach Anspruch 1, wobei ein Nadelkopf der Punktionsnadel (22) eine segmentierte Struktur hat und ein erstes Nadelsegment und ein zweites Nadelsegment aufweist, wobei das erste Nadelsegment eine erste Verlängerungsfläche (221) hat und der zweite Nadelabschnitt eine zweite Verlängerungsfläche (222) hat; eine longitudinale Länge des ersten Nadelsegments kleiner oder gleich einer longitudinalen Länge des zweiten Nadelsegments ist, und ein Winkel, der zwischen einer longitudinalen Mittellinie der ersten Verlängerungsfläche (221) und einer horizontalen Ebene gebildet ist, größer als ein Winkel ist, der zwischen einer longitudinalen Mittellinie der zweiten Verlängerungsfläche (222) und der horizontalen Ebene gebildet ist.

4. Nahtvorrichtung nach Anspruch 1, die ferner einen Bestandteil zur Steuerung der Punktionsnadel aufweist, wobei der Bestandteil zur Steuerung der Punktionsnadel einen Knopf (31), ein Zahnrad (32) und eine Zahnstange (33) aufweist, wobei die Zahnstange (33) mit der Punktionsnadel (22) verbunden ist und der Knopf (31) die Zahnstange (33) durch das Zahnrad (32) in Bewegung setzt.

5. Nahtvorrichtung nach Anspruch 1 oder Anspruch 4, die ferner einen ersten Bestandteil zur Steuerung der Drucknadel und einen zweiten Bestandteil zur Steuerung der Drucknadel aufweist;
der erste Bestandteil zur Steuerung der Drucknadel einen ersten Schieber (41), einen ersten Druckknopf (42) und eine erste Gleitschiene (43) aufweist, die an dem ersten Schieber (41) angeordnet ist, und der erste Druckknopf (42) mit der ersten Drucknadel (23) verbunden ist;
der zweite Bestandteil zur Steuerung der Drucknadel einen zweiten Schieber (51), einen zweiten Druckknopf (52) und eine zweite Gleitschiene (53) aufweist, die an dem zweiten Schieber (51) angeordnet ist, und der zweite Druckknopf mit der zweiten Drucknadel (24) verbunden ist.

6. Nahtvorrichtung nach Anspruch 5, wobei die erste Gleitschiene (43) und die zweite Gleitschiene (53) mit elastischen Bestandteilen zur schnellen Rückführung des ersten Druckknopfes (42) bzw. des zweiten Druckknopfes (52) in entsprechende Ausgangspositionen versehen sind.

7. Nahtvorrichtung nach Anspruch 1, wobei ferner auf einer Seite des Nahthakenstabs (21) eine Nut (29) vorgesehen ist und die Nut (29) der ersten Öffnung entspricht.

8. Nahtvorrichtung nach Anspruch 7, wobei die Nahtanordnung ferner ein Nahtrohr (25) aufweist und das Nahtrohr (25) hinter der Nut (29) angeordnet ist.

9. Nahtvorrichtung nach Anspruch 7, wobei die Nahtanordnung ferner ein Aufnahmerohr (28) aufweist, das Aufnahmerohr (28) in dem Nahthakenstab (21) angeordnet ist und zur Aufnahme der Punktionsnadel (22) aus einer hohlen röhrenförmigen Struktur ausgebildet ist und an einer Seite des Aufnahmerohrs eine zweite Öffnung vorgesehen ist; die Nut (29) und die erste Öffnung der zweiten Öffnung entsprechen.

10. Nahtvorrichtung nach Anspruch 1, wobei am Gehäuse (1) ein Griff (1) vorgesehen ist.

## Revendications

1. Dispositif de suture pour un canal profond, comprenant une enveloppe (1) et un ensemble suture ; dans lequel l'ensemble suture comprend une tige (21) d'accrochage de suture, une aiguille (22) pour ponction, une première aiguille (23) de poussée et une deuxième aiguille (24) de poussée ;
dans lequel la tige (21) d'accrochage de suture présente une structure creuse avec deux extrémités ouvertes, une extrémité supérieure de la tige d'accrochage de suture est dotée d'une partie (26) de courbure, la partie (26) de courbure est en forme de L inversé, et une partie transversale d'une extrémité supérieure de la partie de courbure est dotée d'un trou (27) pour permettre le passage de l'aiguille (22) pour ponction ;
l'aiguille (22) pour ponction présente une structure tubulaire creuse avec une extrémité supérieure ouverte, et un côté de l'aiguille (22) pour ponction est en outre doté d'une première ouverture ;
l'aiguille (22) pour ponction est agencée dans la structure creuse de la tige (21) d'accrochage de suture ;
la première aiguille (23) de poussée et la deuxième aiguille (24) de poussée sont toutes deux agencées dans la structure tubulaire creuse de l'aiguille (22) pour ponction, la première aiguille (23) de poussée est configurée pour pousser la première tige de suture, et la deuxième aiguille (24) de poussée est configurée pour pousser la deuxième tige de suture.

2. Dispositif de suture selon la revendication 1, dans lequel la deuxième aiguille (24) de poussée présente une structure tubulaire creuse, et une extrémité supérieure de la deuxième aiguille de poussée est configurée pour pousser une deuxième tige de suture, et la deuxième tige de suture présente une structure tubulaire creuse ;
la première aiguille (23) de poussée est insérée dans les structures creuses de la deuxième aiguille (24) de poussée et la deuxième tige de suture, et une extrémité supérieure de la première aiguille de poussée est configurée pour pousser la première tige de suture ; la première tige de suture est disposée devant la deuxième tige de suture, et un diamètre extérieur de la première tige de suture est supérieur à un diamètre intérieur de la deuxième tige de suture pour bloquer l'avancement de la deuxième tige de suture.

3. Dispositif de suture selon la revendication 1, dans lequel une tête d'épingle de l'aiguille (22) pour ponction présente une structure segmentée, et comprend un premier segment d'aiguille et un deuxième segment d'aiguille, le premier segment d'aiguille présente une première surface (221) d'extension, et la deuxième section d'aiguille présente une deuxième surface (222) d'extension ; une longueur longitudinale du premier segment d'aiguille est inférieure ou égale à une longueur longitudinale du deuxième segment d'aiguille, et un angle formé entre un axe longitudinal de la première surface (221) d'extension et un plan horizontal est plus grand qu'un angle formé entre un axe longitudinal de la deuxième surface (222) d'extension et le plan horizontal.

4. Dispositif de suture selon la revendication 1, comprenant en outre un composant de commande d'aiguille pour ponction, le composant de commande d'aiguille pour ponction comprend un bouton (31), un engrenage (32) et un support (33), le support (33) est relié à l'aiguille (22) pour ponction, et le bouton (31) entraîne le support (33) pour qu'il soit déplacé par l'engrenage (32).

5. Dispositif de suture selon la revendication 1 ou la revendication 4, comprenant en outre un premier composant de commande d'aiguille de poussée et un deuxième composant de commande d'aiguille de poussée ;
le premier composant de commande d'aiguille de poussée comprend une première glissière (41), un premier bouton-poussoir (42) et un premier rail coulissant (43) agencé sur la première glissière (41), et le premier bouton-poussoir (42) est relié à la première aiguille (23) de poussée ;
le deuxième composant de commande d'aiguille de poussée comprend une deuxième glissière (51), un deuxième bouton-poussoir (52) et un deuxième rail coulissant (53) agencé sur la deuxième glissière (51), et le deuxième bouton-poussoir est relié à la deuxième aiguille (24) de poussée.

6. Dispositif de suture selon la revendication 5, dans lequel le premier rail coulissant (43) et le deuxième rail coulissant (53) sont dotés de composants élastiques pour ramener rapidement le premier bouton-poussoir (42) et le deuxième bouton-poussoir (52) à des positions initiales correspondantes, respectivement.

7. Dispositif de suture selon la revendication 1, dans lequel une rainure (29) est en outre ménagée sur un côté de la tige (21) d'accrochage de suture, et la rainure (29) correspond à la première ouverture.

8. Dispositif de suture selon la revendication 7, dans lequel l'ensemble de suture comprend en outre un tube (25) de suture, et le tube (25) de suture est agencé derrière la rainure (29).

9. Dispositif de suture selon la revendication 7, dans lequel l'ensemble suture comprend en outre un tube (28) de réception, le tube (28) de réception est agencé dans la tige (21) d'accrochage de suture et présente une structure tubulaire creuse pour loger l'aiguille (22) pour ponction, et une deuxième ouverture est ménagée sur un côté du tube de réception ; la rainure (29) et la première ouverture correspondent à la deuxième ouverture.

10. Dispositif de suture selon la revendication 1, dans lequel une poignée (1) est disposée sur l'enveloppe (1).
